Europäisches Patentamt

European Patent Office

Office européen des brevets

(19)

(11) Numéro de publication: **0 457 665 B1**

(12) **FASCICULE DE BREVET EUROPEEN**

(45) Date de publication de fascicule du brevet: **30.11.94**  (51) Int. Cl.⁵: **C07C 45/48**, C07C 49/00

(21) Numéro de dépôt: **91401234.9**

(22) Date de dépôt: **14.05.91**

(54) **Procédé de condensation catalytique d'acides carboxyliques et/ou de leurs dérivés et son application à la préparation de cétones, alcools, amines et amides.**

(30) Priorité: **14.05.90 FR 9006004**

(43) Date de publication de la demande:
**21.11.91 Bulletin 91/47**

(45) Mention de la délivrance du brevet:
**30.11.94 Bulletin 94/48**

(84) Etats contractants désignés:
**BE CH DE DK ES FR GB IT LI NL SE**

(56) Documents cités:
**DE-B- 1 768 138**
**US-A- 1 988 021**

**CHEMICAL ABSTRACTS, vol. 79, 1973, page 384, résumé no. 104703d, Columbus, Ohio, US; I.E. SOSHINA et al.: "Catalytic properties of crystalline and amorphous alumina silicates in the ketonation of carboxylic acids"**

(73) Titulaire: **CENTRE DE COOPERATION INTER-NATIONAL EN RECHERCHE AGRONOMIOUE POUR LE DEVELOPPEMENT**
**42 rue Scheffer**
**F-75016 Paris (FR)**

(72) Inventeur: **Graille, Jean**
**7, La Capelette**
**F-34750 Villeneuve les Maguelonne (FR)**
Inventeur: **Pioch, Daniel**
**1170 Chemin de Nouau**
**F-34730 Prades Le Lez (FR)**

(74) Mandataire: **Hubert, Philippe et al**
**Cabinet Beau de Loménie**
**158, rue de l'Université**
**F-75340 Paris Cédex 07 (FR)**

Rank Xerox (UK) Business Services
(3.10/3.09/3.3.3)

**Description**

La présente invention a essentiellement pour objet un procédé de condensation catalytique d'acides carboxyliques et/ou de leurs dérivés et son application à la préparation de cétones, alcools, amines et amides.

Par "dérivés", on entend désigner ici les esters, halogénures, anhydrides et sels (ou savons) notamment des métaux alcalins et alcalino-terreux ainsi que d'ammonium, d'acides carboxyliques.

L'invention trouve notamment application en oléochimie, pour la préparation de cétones, notamment de cétones aliphatiques supérieures utiles dans des domaines variés. Outre les cétones, ce procédé permet également de préparer, par de simples adaptations des conditions réactionnelles, d'autres composés tels que notamment des alcools, amines, amides, thiocétones et thioalcools.

Il existe actuellement plusieurs procédés de condensation cétonique d'acides carboxyliques ou de leurs dérivés.

La pyrolyse sous pression réduite des sels de métaux alcalins ou alcalino-terreux des acides carboxyliques est connue depuis très longtemps (C. Friedel, Justus Liebig Ann. Chem., 108, 122-128, (1858)). Cependant, ce procédé est peu sélectif, notamment en série aliphatique et conduit à des sous-produits indésirables tels que des hydrocarbures ou des anhydrides d'acides.

Ce procédé n'est donc pas intéressant d'un point de vue industriel.

C'est pourquoi on s'est orienté vers des procédés de condensation catalytique et de très nombreux composés ont été proposés, en tant que catalyseurs.

Par exemple, le document FR-A-2 038 732 décrit un procédé de préparation de cétones aliphatiques supérieures par cétonisation d'acides gras, en phase vapeur, en présence d'un catalyseur constitué par le dioxyde de zirconium ou un système d'oxydes d'aluminium et de zirconium.

On sait également, notamment par les documents GB-1 074 265 et DE-2 758 113, que la transformation des acides ou des esters en cétones peut être catalysée par le carbonate de calcium ou par les oxydes de métaux de transition, notamment les oxydes de zirconium et de thorium.

Le document EP-0 251 111 décrit généralement la préparation de cétones cycliques ayant 4 ou 5 atomes de carbone par condensation d'esters de diacide-carboxyliques, en phage gazeuse, en présence d'oxydes ou de mélange d'oxydes des éléments des groupes IA à VA et IB à VIIB de la classification périodique des éléments.Les oxydes de magnésium, d'aluminium, de silicium, de zinc et de titane sont présentés comme préférentiels pour cette condensation et parmi ceux-ci l'oxyde d'aluminium semble particulièrement intéressant.

Le brevet FR-740 494 décrit généralement un procédé pour la préparation de cétones non saturées par condensation catalytique en phase vapeur d'acides carboxyliques non saturés, ou de leurs dérivés. Il est indiqué que les catalyseurs employés sont de préférence les oxydes métalliques difficilement réductibles et en particulier ceux du calcium, du baryum, du manganèse, du chrome, du titane, du zinc, de l'aluminium et du strontium, ainsi que leurs mélanges. Parmi ces oxydes, l'oxyde de manganèse semble particulièrement intéressant, notamment pour la condensation d'acides gras et en particulier de l'acide oléique.

Malgré leur grand intérêt, notamment comme intermédiaires de synthèse de composés à condensation élevée en carbone, les cétones n'ont été fabriquées jusqu'à présent qu'en faible quantité dans le monde, bien que de très nombreux procédés de préparation ont été proposés dans l'état de la technique.

Ceci peut s'expliquer en partie par le fait que les catalyseurs présentés comme étant les plus performants sont des produits de synthèse qui nécessitent donc une élaboration préalable à l'aide de produits chimiques coûteux.

En outre, l'élaboration de ces produits de synthèse conduit inévitablement à des nuisances pour l'environnement.

Par ailleurs, les procédés décrits dans l'état de la technique sont généralement difficiles à mettre en oeuvre à l'échelle industrielle.

Par exemple, le procédé décrit dans le brevet FR-740 494 nécessite le passage en phase vapeur, le cas échéant avec un gaz vecteur, et dans le cas de produits peu volatils, comme par exemple des acides gras, il s'avère nécessaire de travailler sous pression réduite. De même, le procédé décrit dans le document EP-0 251 111 requiert une réaction en phase gazeuse et l'utilisation d'un gaz vecteur ainsi que l'addition d'eau et de méthanol pour obtenir un rendement élevé.

Il est encore à noter que ces procédés connus ne s'appliquent qu'à certains types bien particuliers de substrats (acides à condenser) à savoir les acides carboxyliques non saturés dans le cas du brevet FR-740 494 et les esters de diacides carboxyliques en C6 dans le cas du document EP-0 251 111.

Enfin, ces procédés présentent des rendements en produits bruts et des sélectivités en composés cétoniques variables, parfois faibles, ce qui nécessite des étapes ultérieures de purification des produits longues et délicates.

En raison des inconvénients précités, les dérivés cétoniques trouvent actuellement l'essentiel de leurs applications comme composants ou additifs de produits à haute valeur comme les arômes ou les parfums quine requièrent que de faibles quantités de ces dérivés.

La présente invention a donc pour but de résoudre le problème technique consistant en la fourniture d'un procédé de condensation catalytique d'acides carboxyliques et/ou de leurs dérivés qui soit d'une mise en oeuvre aisée et adaptée à une production à l'échelle industrielle, qui permette d'obtenir pour une large variété de produits à condenser de bons rendements en produits finals avec une sélectivité élevée et qui utilise un catalyseur peu coûteux, facile à élaborer dans des conditions ne nuisant pas à l'environnement.

La solution, conforme à la présente invention, pour résoudre ce problème technique consiste en un procédé du type précité, caractérisé en ce qu'il comprend la mise en contact à chaud, de préférence à une température comprise entre 350 °C et 450 °C, d'au moins un acide carboxylique ayant au moins 2 atomes de carbone avec un catalyseur à base de composé minéral d'origine naturelle constitué essentiellement par un mélange d'oxydes et/ou d'hydroxydes de fer et d'aluminium, et éventuellement d'oxydes et/ou d'hydroxydes de silicium et de titane et/ou un ou plusieurs composés métalliques choisis parmi le groupe constitué de Cr, Ca, Cu, Co, Zn, Mg, Mn, K, Na, Pb, Ni, comme par exemple un minerai de type latérite ou bauxite, et dont la teneur en poids, en composés du fer est comprise entre 14 et 45 %, de préférence entre 20 et 40 %.

L'expression "constitué essentiellement" signifie que les composés du fer et de l'aluminium présents dans le minerai représentent au moins environ 50 %, et de préférence au moins 65 % en poids, du poids du composé minéral d'origine naturelle calciné à 600 °C.

Dans le cas actuellement préféré où le composé minéral est une bauxite, les composés de fer, d'aluminium, de silicium et de titane représentent de préférence d'environ 90 à environ 100 % en poids du poids dudit composé minéral calciné à 600 °C.

De préférence, on utilise selon l'invention comme catalyseur une bauxite riche en fer, comportant de 14 à 45 % et de préférence de 20 à 40 % en poids de composés du fer.

Il a été découvert, de façon tout à fait surprenante et inattendue, que certains minéraux extraits du sol ou du sous-sol, comme les bauxites et les latérites riches en fer constituent des catalyseurs remarquables pour la réaction de condensation d'une très large variété d'acides carboxyliques. Le faible coût de ces minérais représente un avantage essentiel pour la transformation industrielle par condensation des acides organiques.

Il convient de remarquer que si les propriétés catalytiques d'autres minéraux naturels, comme par exemple les zéolithes ou les argiles ont fait l'objet d'investigations poussées depuis de nombreuses années, il n'en est pas de même pour les bauxites et les latérites.

Il convient en outre de noter que la bauxite est mentionnée comme exemple de catalyseur dans le brevet FR-740 494 discuté précédemment. Cependant, on ne trouve dans ce document antérieur aucune indication sur la teneur en fer de la bauxite utilisée et donc aucune incitation à utiliser une bauxite riche en fer.

Or, il a été découvert, de façon tout à fait inattendue, et ceci constitue le fondement de la présente invention, que les oxydes et hydroxydes de fer, lorsqu'ils sont présents en des proportions en poids d'environ 14 à 45 %, et de préférence de 20 à 40 %, possèdent une activité catalytique plus élevée que celle d'une bauxite telle qu'utilisée normalement pour la fabrication de l'aluminium.

Il a en effet été montré que l'activité catalytique (dans la réaction de condensation envisagée) est reliée à la teneur en composés du fer.

Ainsi, les catalyseurs à base de bauxite riche en fer, selon l'invention, permettent d'obtenir un taux de conversion bien meilleur que celui d'une bauxite à teneur normale (inférieure à 8 % en poids) en composés du fer.

De même, il a été observé de façon tout à fait surprenante que la sélectivité en poids des bauxites en cétones

$$(\text{définie par le rapport} : \frac{\text{cétones formées}}{\text{cétones formées} + \text{autres produits finaux}} \times 100)$$

variait suivant la teneur en composés du fer.

Par exemple, dans le cas de la réaction de condensation de l'acide laurique (qui constitue un composé représentatif des acides susceptibles d'être condensés conformément à l'invention) il a été observé dans des conditions données de taux de conversion (90 %) et de débit de substrat par unité de volume de catalyseur et par heure (v.v.h = 0,3 h⁻¹) que la sélectivité en cétone obtenue était très bonne (au moins égale à 75 %) lorsque la teneur en composés du fer de la bauxite utilisée était comprise entre 14 et 45 % en poids et excellente (d'au moins environ 90 %) lorsque cette teneur était comprise entre 20 et 40 % en poids.

A titre de comparaison, dans les mêmes conditions, la sélectivité en cétone est d'environ 50 % pour une bauxite comportant 8 % en poids de composés du fer.

Il est important pour une application industrielle de travailler dans des conditions garantissant une sélectivité élevée. On peut ainsi limiter, voire même éviter, des traitements subséquents de purification des produits obtenus et diminuer par conséquent leurs prix de revient.

Il n'était pas évident, pour l'homme de l'art, que les catalyseurs à base de bauxite ou de latérite riche en fer possèdent des propriétés catalytiques intéressantes d'autant plus que l'on sait que le fer favorise les réactions de polymèrisation et conduit par conséquent à des rendements en cétones extrêmement peu satisfaisants comme ceci est d'ailleurs rappelé dès le premier paragraphe dans le brevet FR-740 494.

La zone optimale de teneurs en fer, mise en évidence par les inventeurs, semble correspondre à l'existence de sites catalytiques stables à activité élevée. Ceci a d'ailleurs été confirmé par l'étude de l'évolution de la teneur en coke déposé sur le catalyseur. Ce phénomène est très net si l'on travaille dans des conditions comparables, c'est-à-dire à un taux de conversion donné, en ajustant la température.

Ceci confirme l'intérêt de ce type de catalyseurs pour une application industrielle.

Selon une caractéristique particulière du procédé conforme à l'invention, le catalyseur utilisé est obtenu par activation, notamment par calcination, du composé minéral.

Plus précisément, ce catalyseur est élaboré par broyage du composé minéral solide, tamisage, lavage à l'eau, séchage et calcination par les méthodes habituelles bien connues de l'homme du métier. La calcination a pour but de déshydrater le solide afin de créer des sites actifs possédant des propriétés acido-basiques, d'ajuster et de stabiliser la force des groupements électrodonneurs et électroaccepteurs, responsables de l'activité catalytique.

Afin d'améliorer les performances catalytiques du catalyseur, l'étape de calcination peut être précédée d'une lixiviation du solide par des solutions d'acides ou de bases minérales. Cette opération a pour but d'éliminer une partie des composés métalliques qui constituent la surface interne du solide poreux, et donc de modifier les propriétés électroniques de cette surface et, par voie de conséquence, l'activité catalytique.

Par ailleurs, l'activité et la sélectivité du catalyseur peuvent être ajustées ou même modifiées par l'apport de métaux ou de composés métalliques à la surface du solide. Il est également possible de soumettre ce catalyseur à des traitements oxydants (oxygène, gaz soufrés) ou réducteurs (hydrogène, amines). Ces divers ajustements et modifications peuvent très facilement être déterminés par l'homme de métier, selon l'objectif fixé.

Le procédé conforme à la présente invention peut être appliqué aux acides carboxyliques possédant au moins 2 atomes de carbone, qu'ils soient d'origine naturelle comme par exemple les acides gras ou synthétiques, tels les produits d'oxydation des paraffines pétrolières. Avantageusement, il sera appliqué aux acides carboxyliques saturés comportant au moins 6 atomes de carbone, et de préférence de 10 à 20 atomes de carbone.

Selon l'invention, on peut également transformer des composés polyfonctionnels comme par exemple les acides gras saturés ou insaturés, les diacides, les hydroxyacides ainsi que leurs esters, notamment méthyliques ou éthyliques ou des esters de polyols comme par exemple les triglycérides, les anhydrides et les sels.

La condensation catalytique d'acides carboxyliques conduit généralement à des cétones qui peuvent être symétriques dans le cas où les molécules d'acides sont identiques, ou non symétriques dans le cas où les molécules d'acides sont différentes. Par exemple, on peut obtenir une cétone symétrique (RCOR) à partir d'acides gras (RCO₂H) et une cétone non symétrique à partir d'un mélange d'un acide gras (RCO₂H) et d'anhydride acétique (CH₃COOCOCH₃).

A cet égard, il est à noter que, de façon tout à fait surprenante, les bauxites et latérites riches en fer selon l'invention présentent une sélectivité très élevée en cétone non symétrique. On a ainsi observé une très grande sélectivité des bauxites riches en fer pour la fabrication de méthylundécylcétone.

D'une façon générale, le procédé conforme à l'invention peut être également appliqué à la préparation de composés hétéro-atomiques variés, comme les alcools, amines, amides, thiocétones, thioalcools, selon que le produit à transformer est un produit pur ou un mélange et que l'atmosphère sous laquelle la réaction est effectuée est neutre ou rendue oxydante ou réductrice par l'utilisation d'un agent additionnel. Un tel agent peut être par exemple l'hydrogène, l'ammoniac ou une amine à faible condensation en carbone.

Le procédé conforme à l'invention permet donc d'obtenir des produits très divers aux propriétés physico-chimiques variées. Ainsi, à partir d'acide acétique, on peut obtenir de l'acétone, qui est un solvant organique hydrophile. A l'opposé, à partir d'acide stéarique, on peut obtenir la stéarone, qui est hydrophobe et possède un point de fusion relativement élevé.

Pour illustrer cet aspect avantageux de l'invention, on a regroupé au tableau I ci-après les propriétés de trois cétones différentes susceptibles d'être obtenues par la mise en oeuvre du procédé de condensation conforme à l'invention.

TABLEAU I

| SUBSTRAT | PRODUIT | | | |
|---|---|---|---|---|
| Acide carboxylique | Cétone | Poids moléculaire (g) | Temp. de fusion, °C | Temp. d'ébullition °C sous mmHg |
| acétique $CH_3$-COOH | 2-propanone ou acétone $CH_3$-CO-$CH_3$ | 58 | -95 | 56 sous 760 |
| pélargonique $CH_3(CH_2)_7$-COOH | 9-heptadécanone ou pélargone $[CH_3(CH_2)_7]_2CO$ | 254 | 53 | 250 sous 760 142 sous 1 |
| stéarique $CH_3(CH_2)_{16}COOH$ | 18-pentatriacontanone ou stéarone $[CH_3(CH_2)_{16}]_2CO$ | 507 | 88 | décompos. sous 760 |

Le procédé conforme à l'invention peut être mis en oeuvre dans une installation traditionnelle de condensation catalytique, mais ne nécessite pas d'introduire les acides à condenser à l'état de vapeur et ne requiert pas de travailler sous pression réduite. En effet, la pression sera généralement voisine ou égale à la pression ambiante.

Avantageusement, l'acide à transformer sera injecté de façon continue en tête d'un réacteur tubulaire vertical contenant le catalyseur. Eventuellement, cet acide sera préchauffé à une température supérieure à sa température de fusion, avant de passer à travers le lit catalytique.

Ce lit catalytique peut être également surmonté d'un garnissage destiné à ce préchauffage.

Il est à noter que les acides peuvent être introduits avantageusement sans gaz vecteur. Il a en effet été constaté, de façon surprenante, que l'emploi d'un gaz vecteur (par exemple l'azote) préconisé dans l'état de la technique, non seulement n'améliore pas le taux de conversion, mais bien au contraire l'abaisse.

A titre d'exemple, dans le cas de la condensation cétonique d'acides carboxyliques, la réaction peut être effectuée à une température comprise entre environ 250 et 450 °C, de préférence entre environ 340 et 380 °C.

Le débit volumique en acide peut atteindre 2 $h^{-1}$ (2 ml d'acide par ml de catalyseur et par heure). Ce débit doit être adapté à la nature de l'acide à transformer et se situe généralement entre 0,3 et 1 $h^{-1}$.

Les effluents sont condensés et recueillis dans un réservoir situé immédiatement sous le réacteur et porté à une température convenable qui permet de maintenir les produits à l'état liquide tout en évitant de condenser l'eau, co-produit de la réaction. L'eau est généralement entraînée hors du réservoir par les oxydes de carbone qui sont également des co-produits de la réaction.

Le condensat peut être traité à l'aide d'une solution aqueuse d'hydroxyde de sodium, puis lavé à l'eau si son acidité est élevée. Le condensat ainsi traité peut être aussi recristallisé dans l'éthanol afin d'éliminer les dernières traces d'impuretés, en particulier les hydrocarbures et les composés colorés.

Bien entendu, si l'on souhaite obtenir des produits de pureté très élevée, il peut être nécessaire de réaliser une opération complémentaire de distillation sous pression réduite.

D'une façon générale, les acides carboxyliques purs conduisent, par condensation catalytique, à un seul composé cétonique, alors que les mélanges d'acides conduisent à des mélanges de cétones.

Les sous-produits, généralement obtenus en très faibles proportions (inférieures à environ 5 %), sont presque exclusivement des hydrocarbures.

Il convient encore de noter qu'après une utilisation prolongée du catalyseur, qui conduit à sa désactivation partielle, l'activité et la sélectivité initiale peuvent être restaurées par simple réactivation, in situ, dans un courant d'air à une température supérieure à 350 °C, de préférence comprise entre 450 et 600°C.

Les conditions réactionnelles qui viennent d'être décrites sont très différentes de celles imposées dans les procédés de l'état de la technique et notamment ceux décrits dans les documents discutés précédemment. En outre, ces conditions sont parfaitement adaptées à une production industrielle.

On peut également utiliser les catalyseurs riches en fer selon l'invention dans des procédés discontinus, bien qu'un procédé continu soit préférable pour une production industrielle.

Selon un deuxième aspect, la présente invention vise à couvrir un procédé de préparation d'un catalyseur destiné à la condensation d'acides carboxyliques et/ou de leurs dérivés, caractérisé en ce qu'il comprend l'activation par calcination d'un composé minéral d'origine naturelle constitué essentiellement par un mélange d'oxydes et/ou d'hydroxydes de fer et d'aluminium, et éventuellement d'oxydes et/ou d'hydroxydes de silicium et de titane et/ou d'au moins un composé métallique choisi parmi le groupe constitué de Cr, Ca, Cu, Co, Zn, Mg, Mn, K, Na, Pb, Ni, comme par exemple un minerai de type latérite ou bauxite, et dont la teneur en composés du fer est comprise entre 14 et 45 %, de préférence entre 20 et 40 %.

Selon une caractéristique particulière, l'activation comprend le broyage du composé minéral, le tamisage, le lavage à l'eau, le séchage et la calcination.

Avantageusement, préalablement à la calcination, on effectue une lixiviation du composé minéral solide par une solution d'acides ou de bases minérales.

Enfin, selon un troisième aspect, la présente invention vise à couvrir l'utilisation d'un composé minéral d'origine naturelle tel que défini précédemment, pour la fabrication d'un catalyseur destiné à la condensation d'acides carboxyliques et/ou de leurs dérivés.

D'autres buts, caractéristiques et avantages de l'invention apparaîtront plus clairement à la lecture des exemples illustratifs donnés ci-après, quine sauraient limiter la portée de l'invention.

Exemple 1 : Préparation du catalyseur

On a préparé un catalyseur à partir d'une bauxite riche en fer dont la composition en poids est la suivante.

```
Composés :

du fer (par exemple Fe₂O₃, FeO)              31 %

de l'aluminium (par exemple Al₂O₃)           38 %

du silicium (par exemple SiO₂)               12 %

du titane (par exemple TiO₂)                  2 %

du chrome                                    0,15 %

du calcium                                   0,1 %

du magnésium                                 0,06 %

du nickel                                    0,01 %

du manganèse                                 0,01 %

du potassium et du sodium                    0,01 %

du cobalt                                    0,007 %

du zinc                                      0,006 %



du cuivre                                    0,003 %

du plomb                                     0,002 %

eau                                          15 %
```

La bauxite (provenant des Baux-de-Provence) est concassée grossièrement à l'aide d'un marteau puis broyée plus finement jusqu'à une taille moyenne de particules inférieures à un millimètre. La bauxite est ensuite tamisée à l'aide de deux tamis superposés de dimensions de maille 0,05 et 0,5 mm et la fraction retenue par le tamis inférieur de granulométrie comprise entre 0,05 et 0,5 mm est introduite dans un erlenmeyer rempli d'eau déminéralisée. Le volume de l'eau est égal à environ 5 fois le volume de la bauxite. Le contenu de l'erlenmeyer est agité modérément puis laissé décanter et l'eau est finalement séparée par simple égouttage. Cette opération qui a pour but d'éliminer les particules fines pouvant endommager le réacteur est répétée une fois.

L'eau extraparticulaire du solide est laissée évaporer à la température ambiante, puis le séchage est poursuivi à 100°C dans une étuve ventilée pendant 12 h. La calcination est effectuée juste avant la condensation cétonique dans le réacteur lui-même à 600°C pendant deux heures sous courant d'air de 100 ml/min, sur un volume de catalyseur de 15 ml. La calcination constitue l'étape d'activation du solide qui possède alors les propriétés catalytiques requises.

La composition pondérale du catalyseur ainsi activé pour les quatre principaux constituants est alors la suivante : $Fe_2O_3$ 36% ; $Al_2O_3$ 45% ; $SiO_2$ 14% ; $TiO_2$ 2,2%. Il possède une surface spécifique de l'ordre de 65 $m^2$/g.

### Exemple 2

Un volume de 82 ml (69,5 g ; 0,35 mole) d'acide laurique pur est injecté dans un réacteur porté à 380°C, avec un débit volumique de 0,80 $h^{-1}$.

Le réacteur est un tube vertical en silice fondue de diamètre intérieur de 26 mm, équipé d'un "doigt de gant" destiné à recevoir un thermocouple pour mesurer la température du lit catalytique. Ce dernier mesure environ 28 mm de hauteur ; il est retenu à sa base par un fritté (porosité 0) et est surmonté d'un lit de billes de silice fondue de 40 mm de hauteur destiné au préchauffage du substrat à transformer.

Le réacteur, placé dans un four tubulaire thermorégulé, est obturé à sa partie supérieure par un bouchon équipé de deux tubes. Ces derniers permettent respectivement l'introduction d'un gaz (air pour la calcination par exemple) et l'introduction du substrat au moyen d'une pompe. La partie inférieure du réacteur est reliée à un condenseur constitué par un réfrigérant et un ballon récepteur. Le condenseur est maintenu à 80°C par circulation d'eau chaude dans une double enveloppe et le ballon récepteur est relié à l'évent afin d'éliminer les gaz produits par la réaction de condensation (dioxyde de carbone et vapeur d'eau par exemple).

Le condensat liquide jaune paille à 80°C ne présente pas de phase aqueuse. Il pèse 54 g, ce qui représente un rendement en laurone de 92 % par rapport au rendement théorique.

L'analyse chromatographique en phase gazeuse du condensat brut donne la composition centésimale pondérale suivante : laurone 97 ; acide laurique 0,6 ; n-undécane 0,2 ; autres 2,2.

La laurone brute ainsi obtenue possède donc une pureté élevée et pourrait être utilisée telle quelle pour des applications industrielles.

Un test catalytique préalable effectué avec le réacteur contenant un volume de 15 ml de billes de quartz en remplacement du catalyseur a conduit dans les conditions ci-dessus à une phase organique condensée exempte de cétones essentiellement constituée par de l'acide laurique et par une faible proportion d'hydrocarbures.

### Exemple 3

Le lit catalytique précédemment utilisé dans l'exemple 2, traversé par un courant d'air de 60 ml/min, est porté à 600°C pendant 3 h. A la fin de l'opération, le catalyseur est porté à 380°C et 27 ml (23 g ; 0,11 mole) d'acide laurique sont injectés en tête de réacteur avec un débit volumique de 0,80 $h^{-1}$.

La composition centésimale pondérale du condensat est alors la suivante : laurone 98,5 ; acide laurique 0,1 ; n-undécane 0,3 ; autres 1,1.

On constate que l'activité et la sélectivité du catalyseur restent inchangées après avoir transformé prés de 7 fois son volume de substrat.

### Exemple 4

Un volume de 18 ml (15,6 g ; 0,13 mole) d'acide caproïque pur est injecté dans le réacteur porté à 370°C avec un débit volumique de 0,90 $h^{-1}$.

La composition centésimale pondérale de la phase organique brute condensée est la suivante : di n-pentylcétone 91 ; acide caproïque 4 ; autres 5.

Exemple 5

Un volume de 31 ml (38 g ; 0,13 mole) d'un mélange d'acides gras composé principalement de 90% d'acide stéarique et 9 % d'acide palmitique est injecté avec un débit volumique de 1,05 $h^{-1}$ dans le réacteur porté à 385°C.

La composition du condensat est la suivante : $C_{35}H_{70}O$ 67; $C_{33}H_{66}O$ 14 ; $C_{31}H_{62}O$ 1 ; acides gras 7 ; hydrocarbures 8 ; autres 3.

Exemple 6

La phase organique condensée lors du passage de 16 ml (17,9 g ; 0,11 mole) de monoadipate de méthyle sur le catalyseur porté à 350°C avec un débit volumique de 0,4 $h^{-1}$ contient 79 % de cyclopentanone, ce qui correspond à un rendement de 85 % par rapport au rendement théorique.

Exemple 7

La phase organique condensée lors du passage de 18 ml (8,4 g) d'un mélange d'acide laurique (0,02 mole) et d'anhydride acétique (0,04 mole) (1/1 en poids) injecté avec un débit volumique de 0,5 $h^{-1}$ dans le réacteur à 350°C contient après lavage à l'eau 88 % de méthyl n-undécyl cétone ; 5 % d'acide laurique ; 5 % de laurone.

Le procédé de condensation d'acides organiques qui vient d'être décrit et exemplifié présente de nombreux avantages tant sur le plan technique qu'économique, que l'on peut résumer comme suit :
- origine naturelle du catalyseur ;
- faible coût de ce catalyseur ;
- méthode très simple d'activation et de réactivation du catalyseur ;
- température du lit catalytique modérée pour ce type de réaction ;
- activité élevée du catalyseur ;
- possibilité d'application du procédé sur une large gamme d'acides carboxyliques ;
- sélectivité élevée en produits de condensation hétéroatomiques (très faible taux de sous-produits) ;
- produit brut généralement peu coloré ;
- possibilité d'utiliser les produits obtenus, à l'échelle industrielle, sans purification préalable.

**Revendications**

1. Procédé de condensation catalytique d'acides carboxyliques et/ou de leurs dérivés pour la préparation de cétones, alcools, amines, amides, thiocétones, et thioalcools, caractérisé en ce qu'il comprend la mise en contact à chaud, de préférence à une température comprise entre 350°C et 450°C, d'au moins un acide carboxylique ayant au moins 2 atomes de carbone avec un catalyseur à base de composé minéral d'origine naturelle constitué essentiellement par un mélange d'oxydes et/ou d'hydroxydes de fer et d'aluminium, et éventuellement d'oxydes et/ou d'hydroxydes de silicium et de titane et/ou un ou plusieurs composés métalliques choisis parmi le groupe constitué de Cr, Ca, Cu, Co, Zn, Mg, Mn, K, Na, Pb, Ni, comme par exemple un minerai de type latérite ou bauxite, et dont la teneur en poids, en composés du fer est comprise entre 14 et 45 %, de préférence entre 20 et 40 %.

2. Procédé selon la revendication 1, caractérisé en ce que le composé minéral précité est une bauxite riche en fer, comportant de 14 à 45 % et de préférence de 20 à 40 % en poids de composés du fer.

3. Procédé selon la revendication 1 ou 2, caractérisé en ce que le catalyseur précité est obtenu par activation, notamment par calcination, du composé minéral précité.

4. Procédé selon l'une des revendications 1 à 3, caractérisé en ce que l'acide carboxylique précité est un acide carboxylique aliphatique saturé ayant au moins 6 atomes de carbone et de préférence de 10 à 20 atomes de carbone.

5. Procédé selon l'une des revendications 1 à 4, pour la préparation de cétones, caractérisé en ce qu'il consiste à faire passer d'une façon continue, de préférence sans gaz vecteur, le produit à condenser à travers un lit fixe constitué par le catalyseur précité, à une température comprise entre 250°C et 450°C, à une pression voisine ou égale à la pression ambiante.

6. Procédé pour la préparation d'un catalyseur destiné à la condensation d'acides carboxyliques et/ou de leurs dérivés, caractérisé en ce qu'il comprend l'activation par calcination d'un composé minéral d'origine naturelle constitué essentiellement par un mélange d'oxydes et/ou d'hydroxydes de fer et d'aluminium, et éventuellement d'oxydes et/ou d'hydroxydes de silicium et de titane et/ou un ou plusieurs composés métalliques choisis parmi le groupe constitué de Cr, Ca, Cu, Co, Zn, Mg, Mn, K, Na, Pb, Ni, comme par exemple un minerai de type latérite ou bauxite et dont la teneur en poids, en composés du fer est comprise entre 14 et 45 %, de préférence entre 20 et 40 %.

7. Procédé selon la revendication 6, caractérisé en ce que l'activation comprend le broyage du composé minéral, le tamisage, le lavage à l'eau, le séchage et la calcination.

8. Procédé selon la revendication 6 ou 7, caractérisé en ce que, préalablement à la calcination, on effectue une lixiviation du composé minéral solide par une solution d'acides ou de bases minérales.

9. Utilisation d'un composé minéral d'origine naturelle constitué essentiellement par un mélange d'oxydes et/ou d'hydroxydes de fer et d'aluminium, et éventuellement d'oxydes et/ ou d'hydroxydes de silicium et de titane et/ou un ou plusieurs composés métalliques choisis parmi le groupe constitué de Cr, Ca, Cu, Co, Zn, Mg, Mn, K, Na, Pb, Ni, comme par exemple un minerai de type latérite ou bauxite et dont la teneur en poids, en composés du fer est comprise entre 14 et 45 %, de préférence entre 20 et 40 %, pour la préparation d'un catalyseur destiné à la condensation d'acides carboxyliques et/ou de leurs dérivés.

**Claims**

1. Process for the catalytic condensation of carboxylic acids and/or of their derivatives for the preparation of ketones, alcohols, amines, amides, thioketones and thioalcohols, characterized in that it comprises bringing at least one carboxylic acid having at least 2 carbon atoms into contact while hot, preferably at a temperature between 350°C and 450°C, with a catalyst based on an inorganic compound of natural origin consisting essentially of a mixture of oxides and/or of hydroxides of iron and of aluminium, and optionally of oxides and/or of hydroxides of silicon and of titanium and/or one or a number of metal compounds chosen from the group consisting of Cr, Ca, Cu, Co, Zn, Mg, Mn, K, Na, Pb or Ni, such as for example an ore of laterite or bauxite type, and whose content by weight of iron compounds is between 14 and 45%, preferably between 20 and 40%.

2. Process according to Claim 1, characterized in that the abovementioned inorganic compound is an iron-rich bauxite containing from 14 to 45% and preferably from 20 to 40% by weight of iron compounds.

3. Process according to Claim 1 or 2, characterized in that the abovementioned catalyst is obtained by activation, especially by calcination, of the above-mentioned inorganic compound.

4. Process according to one of Claims 1 to 3, characterized in that the abovementioned carboxylic acid is a saturated aliphatic carboxylic acid having at least 6 carbon atoms and preferably from 10 to 20 carbon atoms.

5. Process according to one of Claims 1 to 4, for the preparation of ketones, characterized in that it consists in continuously passing the product to be condensed, preferably without carrier gas, through a stationary bed consisting of the abovementioned catalyst, at a temperature between 250°C and 450°C, at a pressure in the region of or equal to ambient pressure.

6. Process for the preparation of a catalyst intended for the condensation of carboxylic acids and/or of their derivatives, characterized in that it comprises the activation by calcination of an inorganic compound of natural origin consisting essentially of a mixture of oxides and/or of hydroxides of iron and of aluminium, and optionally of oxides and/or of hydroxides of silicon and of titanium and/or one or a number of metal compounds chosen from the group consisting of Cr, Ca, Cu, Co, Zn, Mg, Mn, K, Na, Pb or Ni, such as for example an ore of laterite or bauxite type, and whose content by weight of iron compounds is between 14 and 45%, preferably between 20 and 40%.

7. Process according to Claim 6, characterized in that the activation comprises grinding the inorganic compound, sieving, washing with water, drying and calcination.

8. Process according to Claim 6 or 7, characterized in that, prior to the calcination, the solid inorganic compound is leached with a solution of inorganic bases or acids.

9. Use of an inorganic compound of natural origin consisting essentially of a mixture of oxides and/or of hydroxides of iron and of aluminium, and optionally of oxides and/or of hydroxides of silicon and of titanium and/or one or a number of metal compounds chosen from the group consisting of Cr, Ca, Cu, Co, Zn, Mg, Mn, K, Na, Pb or Ni, such as for example an ore of laterite or bauxite type, and whose content by weight of iron compounds is between 14 and 45%, preferably between 20 and 40%, for the preparation of a catalyst intended for the condensation of carboxylic acids and/or of their derivatives.


**Patentansprüche**


1. Verfahren zur katalytischen Kondensation von Carbonsäuren und/oder ihren Derivaten für die Herstellung von Ketonen, Alkoholen, Aminen, Amiden, Thioketonen und Thioalkoholen,
dadurch **gekennzeichnet,**
daß es die Warmkontaktierung, vorzugsweise bei einer Temperatur von 350 °C bis 450 °C, wenigstens einer Carbonsäure mit wenigstens 2 Kohlenstoffatomen mit einem Katalysator auf Basis einer mineralischen Verbindung natürlichen Ursprungs vorsieht, die im wesentlichen aus einem Gemisch von Oxiden und/oder Hydroxiden von Eisen und Aluminium, und eventuell von Oxiden und/oder Hydroxiden von Silizium und Titan und/oder einer oder mehreren aus der aus Cr, Ca, Cu, Co, Zn, Mg, Mn, K, Na, Pb, Ni gebildeten Gruppe gewählten Metallverbindungen, wie beispielsweise einem Mineral des Laterit- oder Bauxittyps, besteht und deren Gewichtsgehalt an Eisenverbindungen im Bereich von 14 bis 45, vorzugsweise von 20 bis 40 %, liegt.

2. Verfahren nach dem Anspruch 1,
dadurch gekennzeichnet,
daß die vorgenannte mineralische Verbindung ein an Eisen reicher Bauxit ist, der 14 bis 45 und vorzugsweise 20 bis 40 Gew.-% Eisenverbindungen aufweist.

3. Verfahren nach dem Anspruch 1 oder 2,
dadurch gekennzeichnet,
daß der vorgenannte Katalysator durch Aktivierung, insbesondere durch Kalzinierung, der vorgenannten mineralischen Verbindung erhalten wird.

4. Verfahren nach einem der Ansprüche 1 bis 3,
dadurch gekennzeichnet, daß die vorgenannte Carbonsäure eine gesättigte aliphatische Carbonsäure mit wenigstens 6 Kohlenstoffatomen und vorzugsweise 10 bis 20 Kohlenstoffatomen ist.

5. Verfahren nach einem der Ansprüche 1 bis 4, zur Herstellung von Ketonen,
dadurch gekennzeichnet,
daß es darin besteht, das zu kondensierende Produkt kontinuierlich, vorzugsweise ohne Trägergas, durch ein festes, aus dem vorgenannten Katalysator bestehendes Bett bei einer Temperatur im Bereich von 250 °C bis 450 °C sowie bei einem dem Umgebungsdruck nahen oder gleichen Druck strömen zu lassen.

6. Verfahren zur Herstellung eines zur Kondensation von Carbonsäure und/oder ihren Derivaten bestimmten Katalysators,
dadurch gekennzeichnet,
daß es die Aktivierung durch Kalzinierung einer mineralischen Verbindung vorsieht, die im wesentlichen aus einem Gemisch von Oxiden und/oder Hydroxiden von Eisen und Aluminium, und eventuell von Oxiden und/oder Hydroxiden von Silizium und Titan und/oder einer oder mehreren aus der aus Cr, Ca, Cu, Co, Zn, Mg, Mn, K, Na, Pb, Ni gebildeten Gruppe gewählten Metallverbindungen, wie beispielsweise einem Mineral des Laterit- oder Bauxittyps, besteht und deren Gewichtsgehalt an Eisenverbindungen im Bereich von 14 bis 45, vorzugsweise von 20 bis 40 %, liegt.

7.  Verfahren nach dem Anspruch 6,
    dadurch gekennzeichnet,
    daß die Aktivierung die Feinzerkleinerung der mineralischen Verbindung, die Siebung, das Waschen mit Wasser, die Trocknung und die Kalzinierung umfaßt.

8.  Verfahren nach dem Anspruch 6 oder 7,
    dadurch gekennzeichnet,
    daß man vor der Kalzinierung eine Auslaugung der festen mineralischen Verbindung durch eine Lösung mineralischer Säuren oder Basen vornimmt.

9.  Verwendung einer mineralischen Verbindung natürlichen Ursprungs, die im wesentlichen aus einem Gemisch von Oxiden und/oder Hydroxiden von Eisen und Aluminium, und eventuell von Oxiden und/oder Hydroxiden von Silizium und Titan und/oder einer oder mehreren aus der aus Cr, Ca, Cu, Co, Zn, Mg, Mn, K, Na, Pb, Ni gebildeten Gruppe gewählten Metallverbindungen, wie beispielsweise einem Mineral des Laterit- oder Bauxittyps, besteht und deren Gewichtsgehalt an Eisenverbindungen im Bereich von 14 bis 45, vorzugsweise von 20 bis 40 %, liegt, zur Herstellung eines Katalysators, der zur Kondensation von Carbonsäuren und/oder ihren Derivaten bestimmt ist.